# EUROPEAN PATENT SPECIFICATION

(11) **EP 1 728 463 B1**
(45) Date of publication and mention of the grant of the patent: **25.01.2012**
(21) Application number: 06011509.4
(22) Date of filing: 02.06.2006
(51) Int. Cl.: A61B 1/012, A61B 8/12

(54) **Endoscope systems**
Endoscope Systeme
Systèmes d'endoscope

(30) Priority: 02.06.2005 JP 2005163110
(43) Date of publication of application: 06.12.2006
(73) Proprietor: FUJIFILM Corporation, Minato-ku Tokyo (JP)
(72) Inventor: Sato, Yoshiaki, Kaisei-machi Ashigarakami-gun Kanagawa (JP)
(74) Representative: Klunker . Schmitt-Nilson . Hirsch

(56) References cited:
- WO-A-2005/120329
- US-A- 5 868 666
- US-A1- 2004 141 054
- US-B1- 6 397 286

## Description

### FIELD OF THE INVENTION

The present invention relates to an ultrasonic endoscope system according to the preamble of claim 1 that takes endoscopic images and ultrasonographic images using an ultrasonic endoscope. The present invention also relates to an electronic endoscope system according to the preamble of claim 17 that takes endoscopic images using an electronic endoscope.

### BACKGROUND OF THE INVENTION

Diagnosis utilizing an endoscope has recently been put into practice in the medical field. The endoscope has an imaging device, such as a CCD, at its end, for capturing an optical image of a certain part inside a living body, to take an endoscopic image. Some endoscope is provided with an ultrasonic transducer that projects ultrasonic waves toward a certain internal body part, and receives echo signals from the internal body part, to take an ultrasonographic image, so that ultrasonic diagnosis can be made simultaneously with endoscopic diagnosis. Among those endoscopes, one has a treatment tool introduction channel for introducing an ultrasonic probe having an ultrasonic transducer at its end, and another has an ultrasonic transducer disposed near an imaging device. The latter type is called an ultrasonic endoscope.

When the endoscope having the imaging device and the ultrasonic transducer is used for making endoscopic diagnosis and ultrasonic diagnosis at the same time, a processor for endoscope and a processor for ultrasonograph have been necessary for producing an endoscopic image from the signal captured through the imaging device and for producing an ultrasonographic image from the echo signal. These processors have to be operated individually, and the endoscopic image and the ultrasonographic image are displayed and observed on individual monitors. Accordingly, the conventional system is disadvantageous in view of installation space efficiency and operability.

In order to solve these problems, Japanese Laid-open Patent Application No. Hei 11-309148 suggests an ultrasonic diagnostic apparatus that displays an endoscopic image and an ultrasonic image as a composite image, called a picture-in-picture display, on a common monitor. Japanese Laid-open Patent Application No. 2001-145627 suggests a medical image observer that has a common operational section for an endoscope and an ultrasonic transducer.

Owing to the common monitor or the common operational section, these prior arts are improved in the installation space efficiency and the operability. But these prior arts do not fundamentally solve the above problems.

In accordance with the preamble of claim 1, US-B1-6 397 286 discloses an ultrasonic endoscope system which may have different network configurations each comprising a number of units that are connected via interfaces to a self-configuring bus (such as FireWire or a CAN bus (Controller Area Network)). The document further discloses that bus arbitration and data communication can be implemented in the system. This prior art system comprises a microprocessor, which is connected to peripheral equipment via an RS 232 interface (which does not have bus arbitration capabilities). The CAN bus connects a master computer with further units outside the endoscope device.

### SUMMARY OF THE INVENTION

In view of the foregoing, a primary object of the present invention is to provide such an ultrasonic endoscope system and an electronic endoscope system, of which installation space is less restricted than conventional, and which are remarkably improved in operability.

To solve the above and other objects, an ultrasonic endoscope system of the present invention comprises the features of claim 1.

Preferred embodiments are defined by the dependent claims.

The serial buses connecting the peripheral equipment control unit to the processors and the peripheral equipments are preferably universal serial buses. The peripheral equipment control unit controls those of the universal serial buses which are connected respectively to the processors, independently from each other. The the peripheral equipment control unit comprises at least two USB controllers and a CPU for controlling operation of the USB controllers, and operates as a host while exchanging data with the peripheral equipments, and as a device while exchanging data with the processors.

According to a preferred embodiment, the peripheral equipments include a movie recorder for recording a selected one of the endoscopic image and the ultrasonographic image as a movie image. The peripheral equipments include a first operational device for selecting one of the endoscopic image and the ultrasonographic image, to be recorded by the movie recorder. The processors are preferably connected to the movie recorder through an IEEE1394 bus, wherein an output plug and an input plug of the IEEE1394 bus are established at a first one of the processors, a second output plug of the IEEE1394 bus is established at a second one of the processors, and a second input plug of the IEEE1394 bus is established at the movie recorder, and wherein a first isochronous channel is established between the first processor and the movie recorder, and a second isochronous channel is established between the first and second processors.

According to another preferred embodiment, the ultrasonic endoscope system further comprises an image composing device for composing the endoscopic image and the ultrasonographic image to produce a composite image, and an image display device for displaying a selected one of the endoscopic image, the ultrasonographic image and the composite image.

An electronic endoscope system of the present invention comprises the features of claim 17.

Preferably, a peripheral equipment control unit is connected to the endoscope processor and the peripheral equipments through the serial buses. The peripheral equipment control unit serves as an intermediary of data between the endoscope processor and the peripheral equipments, wherein the peripheral equipments preferably include at least a second processor that produces from an electric signal an image served for medical diagnosis, such as an ultrasonographic image or an optical coherent tomographic image.

According to the present invention, the endoscope processor and the ultrasonograph processor or other kinds of processors can share the same peripheral equipments. Because the processors are connected to the peripheral equipments through the serial buses having bus arbitration function, wiring is simplified in comparison with a conventional system that adopts RS232C-connection or PS/2-connection.

### BRIEF DESCRIPTION OF THE DRAWINGS

The above and other objects and advantages will be more apparent from the following detailed description of the preferred embodiments when read in connection with the accompanied drawings, wherein like reference numerals designate like or corresponding parts throughout the several views, and wherein:
Figure 1 is a block diagram illustrating an ultrasonic endoscope system according to a first embodiment of the present invention;
Figure 2 is a block diagram illustrating a peripheral equipment control unit of the ultrasonic endoscope system;
Figure 3 is a view showing frame format of connection between the peripheral equipment control unit and each of an endoscope processor, an ultrasonograph processor and peripheral equipments through respective USB;
Figure 4 is a view showing frame format of connection between the processors and a movie recorder through an IEEE1394 bus;
Figure 5 is a schematic view of a keyboard of the ultrasonic endoscope system of the first embodiment;
Figure 6 is a flow chart illustrating a sequence for printing data by a printer;
Figure 7 is a flow chart illustrating a sequence for writing data on a storage medium through a driver;
Figure 8 is a flow chart illustrating a sequence of processing responsive to an operational signal entered through a keyboard or a footswitch;
Figure 9 is a flow chart illustrating a sequence for reading data out of a memory card through a card reader;
Figure 10 is a flow chart illustrating a sequence of processing responsive to an image recording signal entered through an endoscopic image recording switch or an ultrasonographic image recording switch;
Figure 11 is a block diagram illustrating an ultrasonic endoscope system according to a second embodiment of the present invention;
Figure 12 is a schematic view of a keyboard of the ultrasonic endoscope system of the second embodiment;
Figure 13 is an explanatory diagram illustrating various display modes selectable by operating a display mode switch or a display mode dial; and
Figure 14 is a block diagram illustrating an electronic endoscope system of the present invention

### DESCRIPTION OF THE PREFERRED EMBODIMENTS

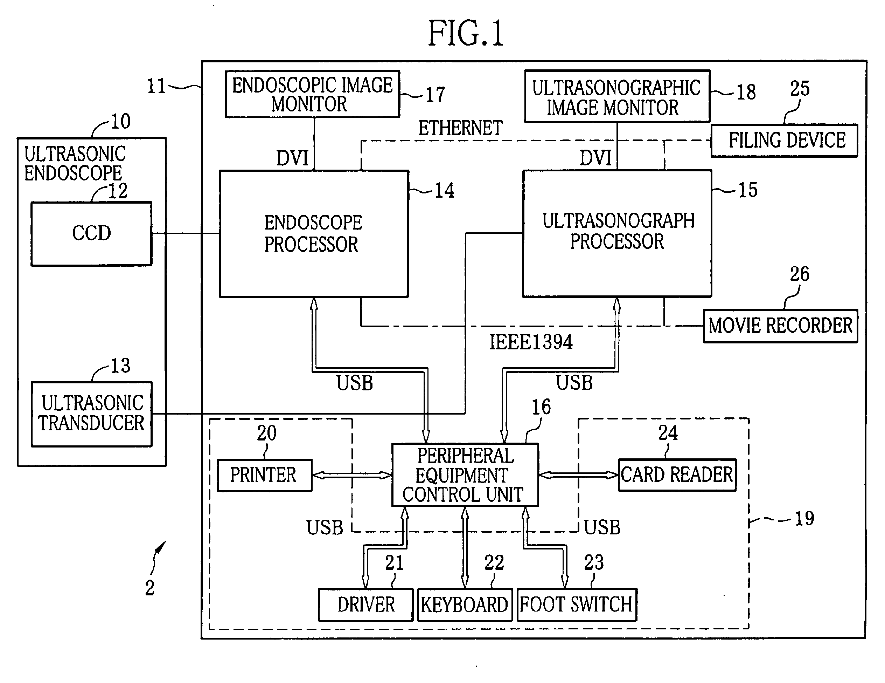

In Fig.1, an ultrasonic endoscope system 2 of the present invention consists of an ultrasonic endoscope 10 and an observation device 11. The ultrasonic endoscope 10 has a CCD 12 and an ultrasonic transducer 13 incorporated therein, wherein the CCD 12 captures an optical image of a certain internal body part to output an image signal, and the ultrasonic transducer 13 emits ultrasonic waves toward the certain internal body part and receives an echo signal from the certain internal body part.

The observation device 11 is mainly constituted of an endoscope processor 14, an ultrasonograph processor 15 and a peripheral equipment control unit 16. The endoscope processor 14 is connected to the CCD 12, to control driving the CCD 12, produce an endoscopic image from the image signal entered by the CCD 12, and output digital data of the endoscopic image to a monitor for endoscopic image 17. The endoscopic image monitor 17 is connected to the endoscope processor 14 through DVI (digital visual interface).

The ultrasonograph processor 15 is connected to the ultrasonic transducer 13, to control driving the ultrasonic transducer 13, produce an ultrasonographic image from the echo signal entered by the ultrasonic transducer 13, and outputs digital data of the ultrasonographic image to a monitor for ultrasonographic image 18. The ultrasonographic image monitor 18 is connected to the ultrasonograph processor 15 through the DVI.

The peripheral equipment control unit 16 is connected to the respective processors 14 and 15 and peripheral equipments 19 through USB (universal serial bus) connections. The peripheral equipments 19 include a printer 20 for printing out the endoscopic image or the ultrasonographic image, and a driver 21 for reading and writing data between the peripheral equipment control unit 16 and external storage media, such as floppy disc (trade name), MO, CD-ROM, DVD-ROM and flash memory. The peripheral equipments 19 further include a keyboard 22, a footswitch 23 and a card reader 24 for reading data out of a memory card that stores personal data of a patient, such as the name and the sex of the patient.

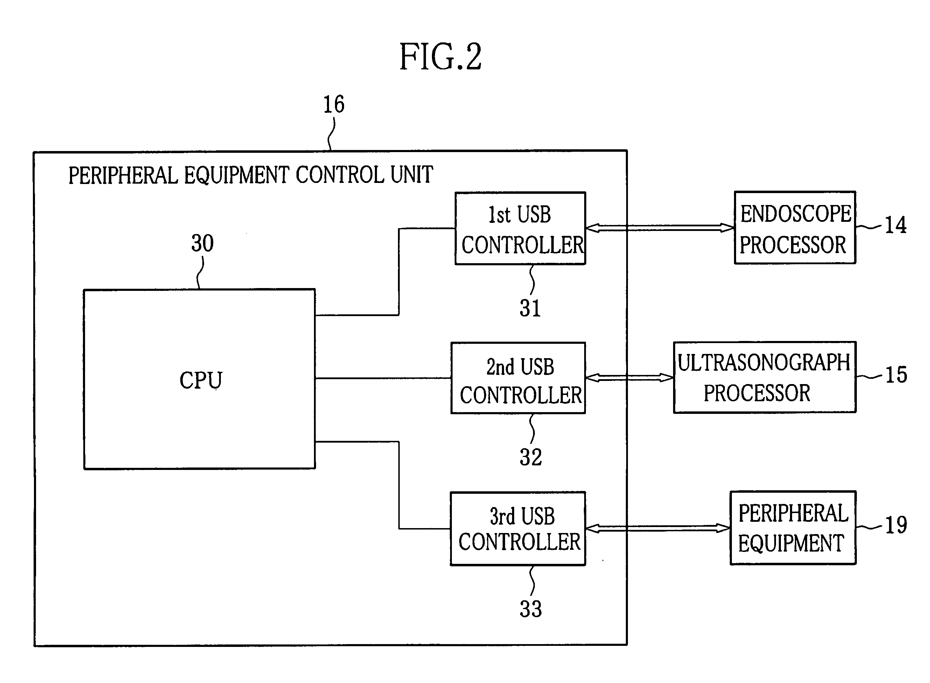

As shown in Fig.2, the peripheral equipment control unit 16 consists of a CPU 30 and first to third USB controllers 31, 32 and 33, which operate under the control of the CPU 30. The first to third USB controllers 31 to 33 are connected to the endoscope processor 14, the ultrasonograph processor 15 and the peripheral equipments 19 respectively. Specifically, the third USB controller 33 has a switching hub function.

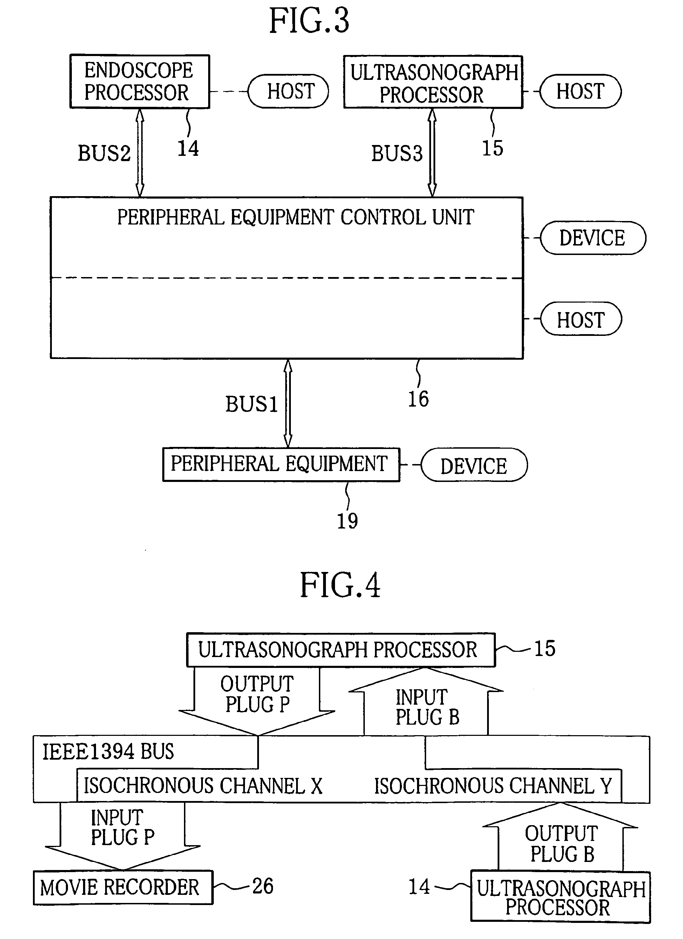

As shown conceptually in Fig.3, the respective processors 14 and 15 and the peripheral equipments 19 operate as hosts and devices to the peripheral equipment control unit 16. The peripheral equipment control unit 16 operates as a host while exchanging data with the peripheral equipments 19 through a bus BUS1, and also operates as a device while exchanging data with the respective processors 14 and 15 through individual buses BUS2 and BUS3. The CPU 30 switches the peripheral equipment control unit 16 between the host operation and the device operation.

Referring back to Fig.1, the respective processors 14 and 15 are connected to a filing device 25 and a movie recorder 26. The filing device 25 is connected to the respective processors 14 and 15 through Ethernet (trade name) as show by dashed line in Fig.1. The filing device 25 obtains the endoscopic image and the ultrasonographic image from the respective processors 14 and 15 through the Ethernet, to record them as image files.

The movie recorder 26 is connected to the respective processors 14 and 15 through IEEE1394 bus as shown by chain-dotted lines in Fig.1. The movie recorder 26 obtains the endoscopic image and the ultrasonographic image from the respective processors 14 and 15 through the IEEE1394 bus, to record them digitally on a recording medium, like a recording tape.

As shown in Fig.4, when the movie recorder 26 is connected to the respective processors 14 and 15 through the IEEE1394 bus, an output plug P (point to point out) and an input plug B (broadcast in) of the IEEE1394 bus are established at the ultrasonograph processor 15, whereas an output plug B (broadcast out) of the IEEE1394 bus is established at the endoscope processor 14, and an input plug P (point to point in) of the IEEE1394 bus is established at the movie recorder 26. Simultaneously, a first isochronous channel X is established between the ultrasonograph processor 15 and the movie recorder 26, whereas a second isochronous channel Y is established between the endoscope processor 14 and the ultrasonograph processor 15.

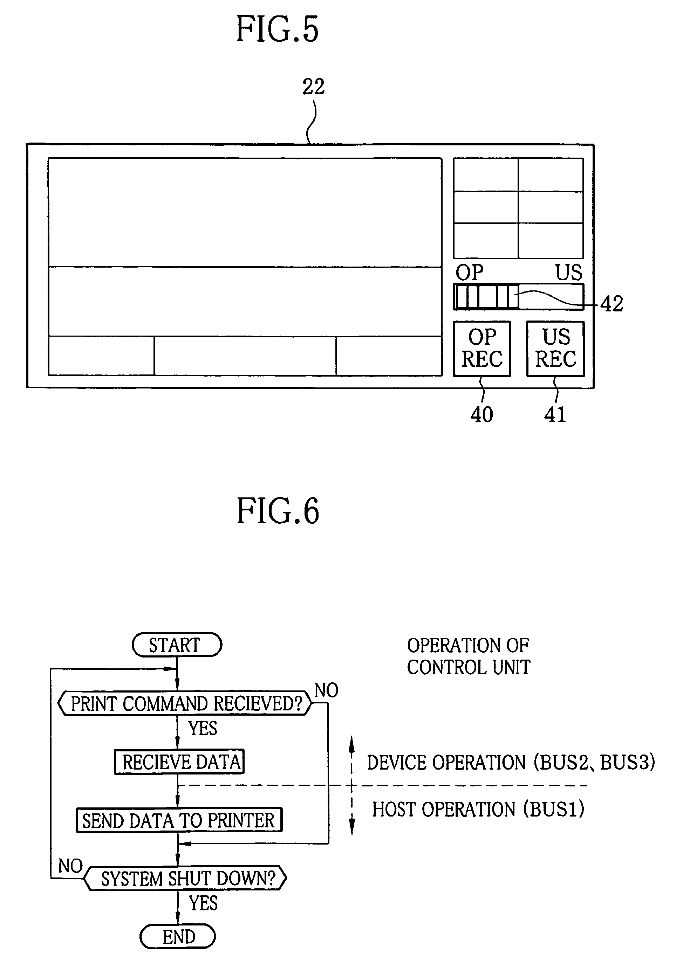

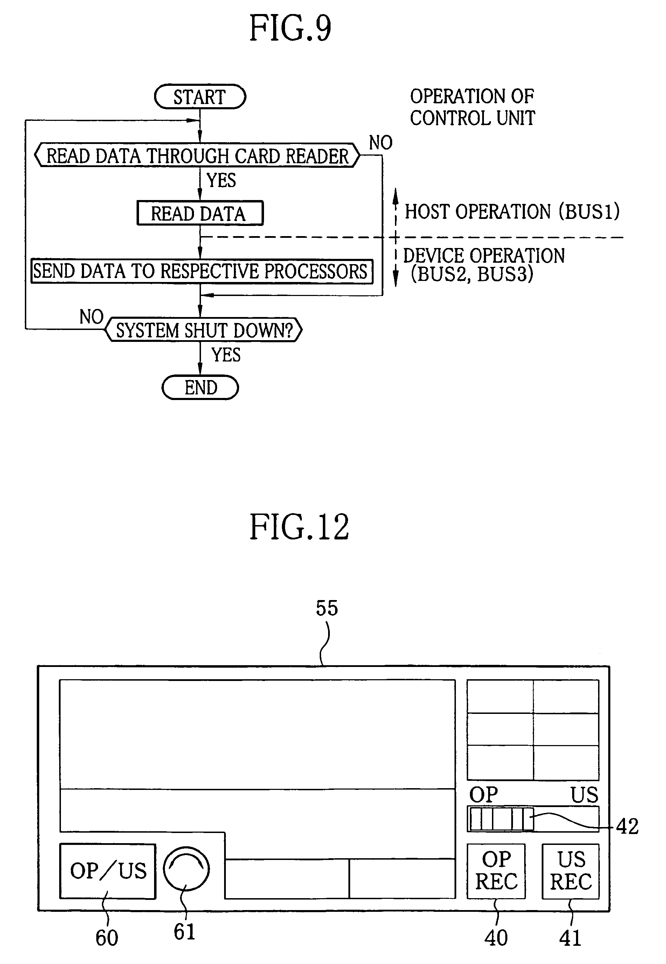

As shown in Fig.5, the keyboard 22 is provided with an endoscopic image recording switch 40, an ultrasonographic image recording switch 41 and a recording priority selection switch 42. The endoscopic image recording switch 40 is operated to record the endoscopic image as a movie image by the movie recorder 26, whereas the ultrasonographic image recording switch 41 is operated to record the ultrasonographic image as a movie image by the movie recorder 26. The recording priority selection switch 42 is operated to choose between an endoscope priority mode for giving priority to the movie recording of the endoscopic image and an ultrasonograph priority mode for giving priority to the movie recording of the ultrasonographic image. In Fig. 5, OP and US stand for endoscopic image and ultrasonographic image respectively, and the same applies to Fig.12.

The endoscope processor 14 controls the movie recorder 26 such that the movie recorder 26 alternately starts and stops the movie recording of the endoscopic image upon the endoscopic image recording switch 40 being operated. The ultrasonograph processor 15 controls the movie recorder 26 such that the movie recorder 26 alternately starts and stops the movie recording of the ultrasonographic image upon the ultrasonographic image recording switch 41 being operated. The respective processors 14 and 15 also control the movie recorder 26 to record either the endoscopic image or the ultrasonographic image, which is selected by the recording priority selection switch 42, prior to the recording as designated by the endoscopic image recording switch 40 or the ultrasonographic image recording switch 41. Concretely, when a command for starting recording a first one of the two kinds of images is entered by operating the image recording switch 40 or 41 while the movie recorder 26 is recording a second one of the two kinds of images, and if at that time the first kind image is selected by the recording priority selection switch 42, the movie recorder 26 interrupts recording the second kind image and starts recording the first kind image. On the contrary, even when the command for starting recording the first kind image is entered, if the second kind image is selected by the recording priority selection switch 42, the movie recorder 26 continues recording the second kind image and does not start recording the first kind image. Instead, a warning like "VTR BUSY" is displayed on the monitor 17 or 18 for the first kind image.

When a command for obtaining an endoscopic image is entered while an inserting portion of the ultrasonic endoscope 10 is inserted in a living body, the CCD 12 captures an optical image of a certain part inside the living body and outputs an image signal. The image signal from the CCD 12 is fed to the endoscope processor 14.

The endoscope processor 14 subjects the image signal to many kinds of image-processing to produce the endoscopic image. The endoscopic image produced through the endoscope processor 14 is displayed on the endoscopic image monitor 17.

While the endoscopic image is being observed on the endoscopic image monitor 17, a particular internal body part is searched. When an end of the ultrasonic endoscope 10 reaches the particular internal body part, and a command for obtaining an ultrasonographic image is entered, the ultrasonic transducer 13 emits the ultrasonic wave toward the particular body part. Then the particular body part reflects the echo signal in correspondence to the ultrasonic wave, so the ultrasonic transducer 13 receives the echo signal. The echo signal received on the ultrasonic transducer 13 is fed to the ultrasonograph processor 15.

The ultrasonograph processor 15 subjects the echo signal to many kinds of image-processing to produce the ultrasonographic image. The ultrasonographic image produced through the ultrasonograph processor 15 is displayed on the ultrasonographic image monitor 18.

Now the operation of the peripheral equipment control unit 16 will be described with reference to Figs.6 to 9.

As shown in Fig.6, when the peripheral equipment control unit 16 receives a print command for the printer 20 from the respective processors 14 and 15, the peripheral equipment control unit 16 operates as a device to the respective processors 14 and 15, on receiving data to print from the respective processors 14 and 15. Then, the peripheral equipment control unit 16 operates as a host to the printer 20, on sending the received data to the printer 20.

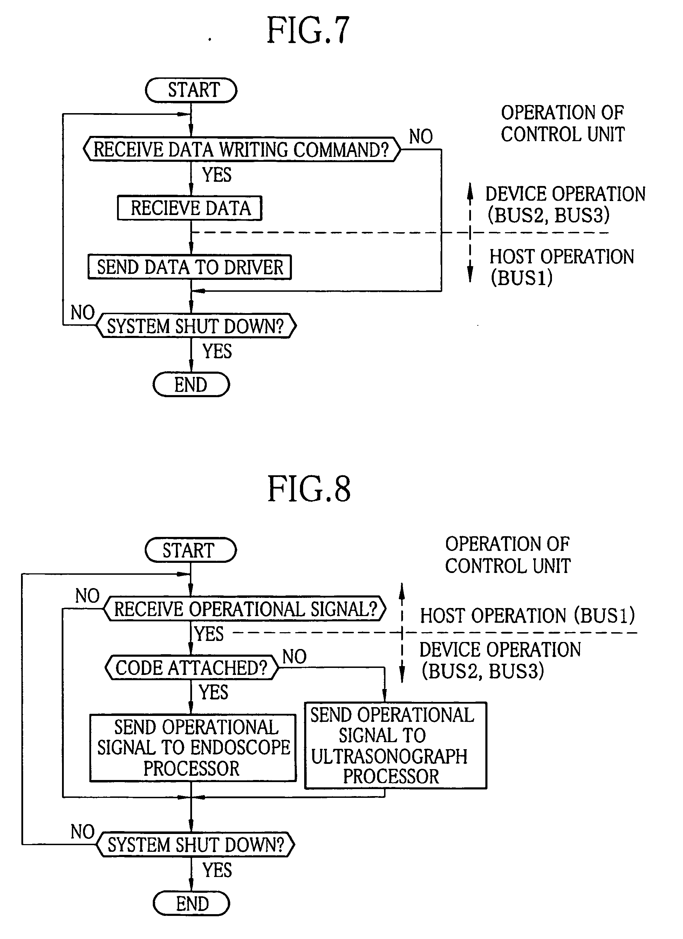

When the peripheral equipment control unit 16 receives a command for writing data on a storage medium from the respective processors 14 and 15, as shown in Fig. 7, the peripheral equipment control unit 16 operates as a device to the respective processors 14 and 15, on receiving the data to write. Then the peripheral equipment control unit 16 operates as a host to the driver 21, on sending the received data to the driver 21.

On the other hand, as shown in Fig.8, the peripheral equipment control unit 16 operates as a device to the keyboard 22 or the footswitch 23, on receiving an operational signal from the keyboard 22 or the footswitch 23. Then the peripheral equipment control unit 16 operates as a host to the respective processors 14 and 15, on sending the received operational signal to the endoscope processor 14 or the ultrasonograph processor 15. In order to distinct the operational signal assigned to the endoscope processor 14 from one assigned to the ultrasonograph processor 15, a specific code is attached to the operational signal for the endoscope processor 14, so that the peripheral equipment control unit 16 checks the operational signal if the specific code is attached or not, to determine which of the processors 14 and 15 the operational signal should be sent to.

When the peripheral equipment control unit 16 reads data through the card reader 24, as shown in Fig.9, the peripheral equipment control unit 16 operates as a host to the card reader 24. Then, on sending the read data to the respective processors 14 and 15, the peripheral equipment control unit 16 operates as a device to the respective processors 14 and 15.

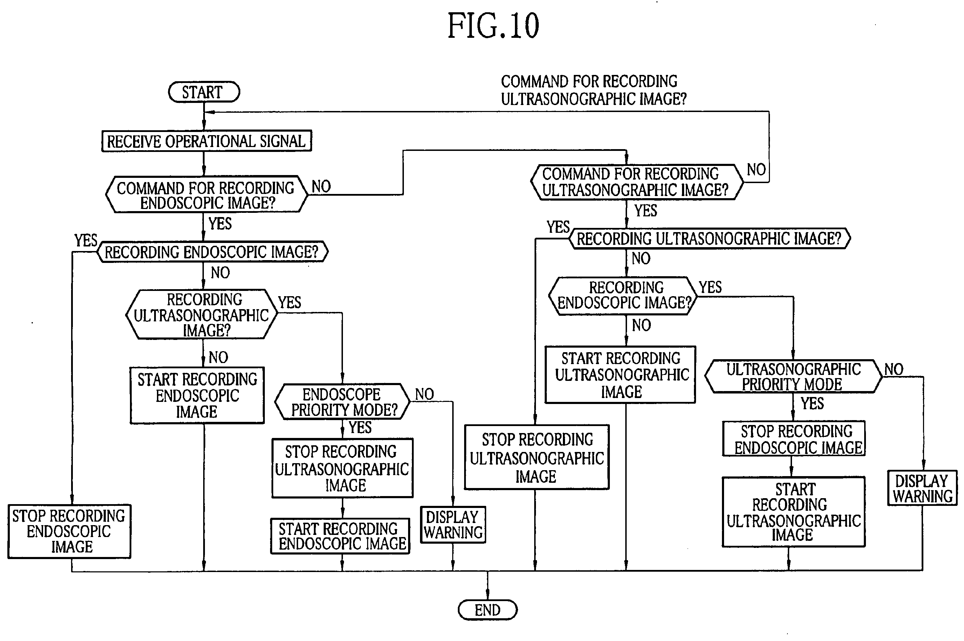

As shown in Fig.10, when an endoscopic image recording command is entered by operating the endoscopic image recording switch 40, is received on the endoscope processor 14 through the peripheral equipment control unit 16, the endoscope processor 14 checks the state of operation of the movie recorder 26. If the movie recorder 26 is already recording the endoscopic image, the endoscope processor 14 sends the movie recorder 26 a command for stopping recording the endoscopic image, so the movie recorder 26 stops the recording of the endoscopic image.

If the movie recorder 26 is not recording the endoscopic image nor the ultrasonographic image, the endoscope processor 14 sends the movie recorder 26 a command for starting recording the endoscopic image, so the movie recorder 26 stars recording the endoscopic image.

If, on the other hand, the movie recorder 26 is recording the ultrasonographic image, and the endoscope priority mode is selected by the recording priority selection switch 42, the endoscope processor 14 sends the movie recorder 26 a command for stopping recording the ultrasonographic image. After the recording of the ultrasonographic image is stopped, the endoscope processor 14 sends the movie recorder 26 the command for starting recording the endoscopic image, so the movie recorder 26 stars recording the endoscopic image.

If the endoscope priority mode is not selected by the recording priority selection switch 42, a warning is displayed on the endoscopic image monitor 17. Note that the operation of the ultrasonograph processor 15 responsive to an ultrasonographic image recording command, which is entered through the ultrasonographic image recording switch 41, is the same as the above-described operation of the endoscope processor 14 responsive to the endoscopic image recording command, if only "the endoscopic image" is replaced with "the ultrasonographic image". So the detailed description relating the ultrasonographic image recording command is omitted.

As set forth above, the endoscope processor 14 and the ultrasonograph processor 15 are connected to the peripheral equipments 19 via the peripheral equipment control unit 16 by the USB connection, whereas the processors 14 and 15 are connected to the filing device 25 and the movie recorder 26 through the Ethernet and the IEEE1394 bus respectively. Accordingly, the endoscope processor 14 and the ultrasonograph processor 15 use the same peripheral equipments 19. Besides that, wiring between the equipments and the devices is simplified in comparison with a conventional ultrasonic endoscope system that adopts RS232C-connection or PS/2-connection.

Since it is possible to choose between the endoscope priority mode where the movie recording of the endoscopic image has priority, on one hand, and the ultrasonograph priority mode where the movie recording of the ultrasonographic image has priority, the system operator can record the image of the designated kind without fail.

As a variation, the peripheral equipment control unit 16 may be built in the endoscope processor 14. It is also possible to integrate the first or the second USB controller 31 or 32 with the third USB controller 33.

In the frame format of connection shown in the Fig.4, the position of the endoscope processor 14 may be exchanged with the position of the ultrasonograph processor 15. The plugs of the second isochronous channel Y may be the point to point type.

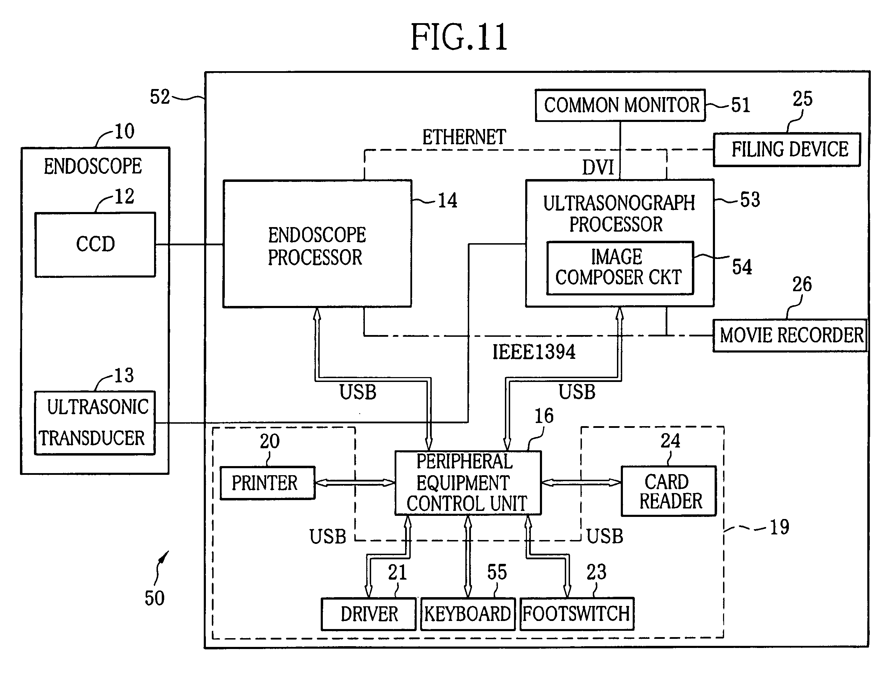

Next, another embodiment of the present invention will be described with reference to Figs.11 to 14, wherein the same or like parts are designated by the same reference numerals, so the following description will relate to merely those parts essential to the second embodiment.

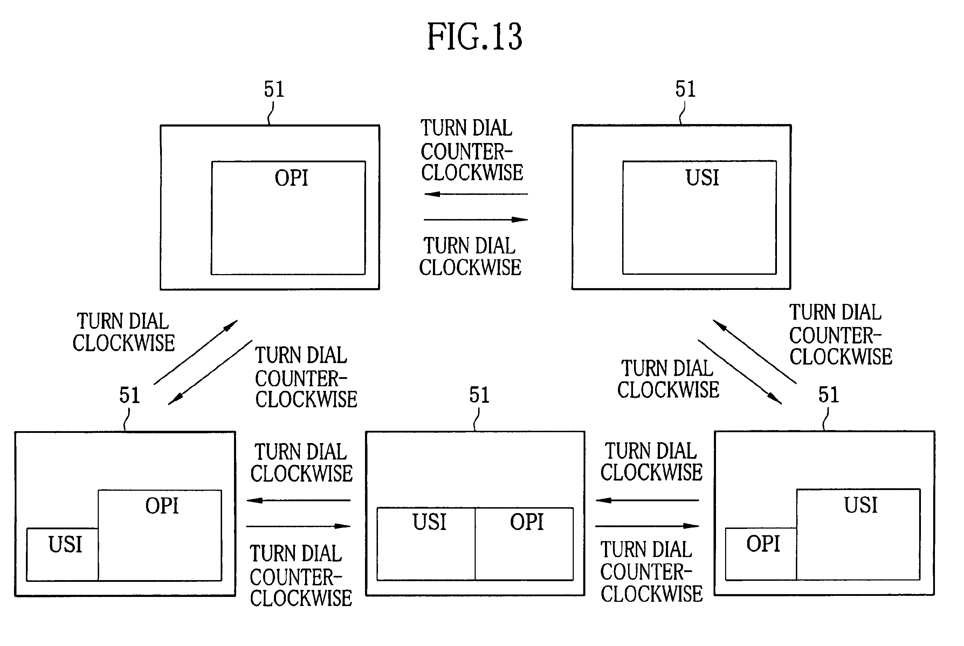

In Fig.11, an ultrasonic endoscope system 50 is provided with an observer 52 having a common monitor 51 that displays an endoscopic image, an ultrasonic image or a composite image as set forth in detail later. The common monitor 51 is connected to an ultrasonograph processor 53. The ultrasonograph processor 53 is provided with an image composer circuit 54 for producing the composite image by composing the endoscopic image, which is sent from an endoscope processor 14 through an IEEE1394 bus, with the ultrasonographic image produced by the ultrasonograph processor 53. The image composer 54 can change the ratio of an endoscopic image display area to an ultrasonographic image display area in the composite image on the screen of the common monitor 51. For example, as shown in Fig.13, the ratio may change in three steps of 4:1, 1:1 and 1:4.

As shown in Fig. 12, a keyboard 55 of the ultrasonic endoscope system 50 has a display mode switch 60 and a display mode dial 61 in addition to image recording switches 40 and 41 and a recording priority selection switch 42. The display mode dial 61 can work only while the display mode switch 60 is turned on. Concretely, it becomes possible to turn the display mode dial 61 when the display mode switch 60 is pressed down.

As shown in Fig.13, as the display mode dial 61 is turned after the display mode switch 60 is pressed down and thus turned on, the display mode on the common monitor 51 changes. In Fig. 13, USI represents the ultrasonographic image, and OPI represents the endoscopic image. Using the common monitor 51 for displaying the endoscopic image and the ultrasonographic image makes the ultrasonic endoscope system 50 still more compact.

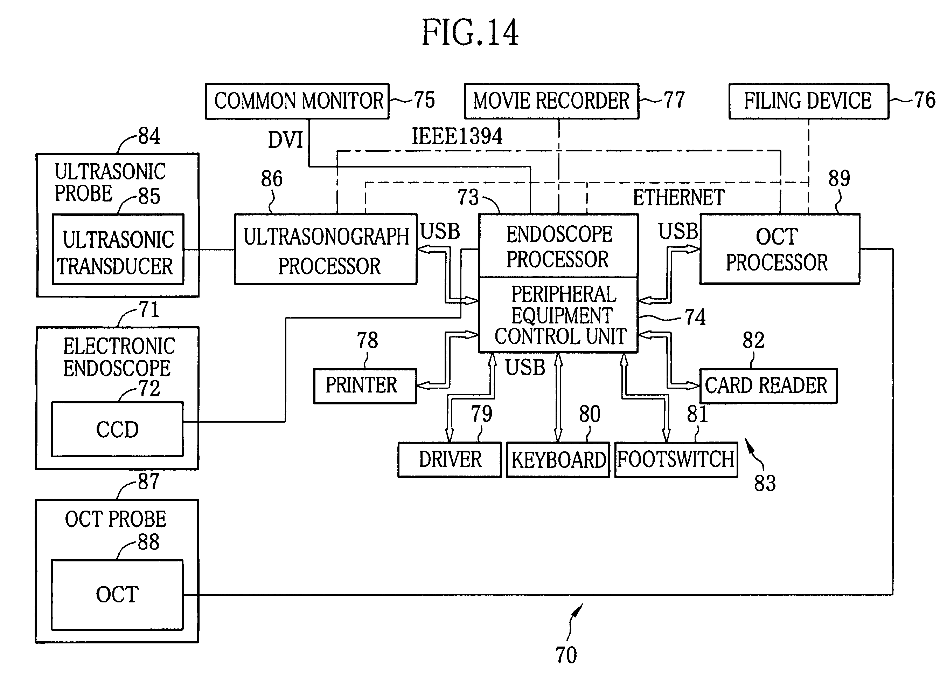

Fig.14 shows an electronic endoscope system 70 of the present invention. The electronic endoscope system 70 consists of an electronic endoscope 71, an endoscope processor 73 that is connected to a CCD 72 of the electronic endoscope 71, and a peripheral equipment control unit 74 that is integrated in the endoscope processor 73. The endoscope processor 73 produces an endoscopic image from an image signal output from the CCD 72.

The endoscope processor 73 is connected to a common monitor 75, a filing device 76 and a movie recorder 77 through the DVI connection, the Ethernet and the IEEE1394 bus, respectively. The peripheral equipment control unit 74 is connected to peripheral equipments 83, including a printer 78, a driver 79, a keyboard 80, a footswitch 81 and a card reader 82, through USB connections.

The peripheral equipment control unit 74 is also connected to an ultrasonograph processor 86 and an OCT (optical coherent tomography) processor 89 through the USB connections. The ultrasonograph processor 86 is connected to an ultrasonic transducer 85 of an ultrasonic probe 84, to produce an ultrasonographic image from an echo signal received on the ultrasonic transducer 85. The OCT processor 89 is connected to an OCT element 88 of an OCT probe 87, to produce an optical coherent tomographic image. The ultrasonograph processor 86 and the OCT processor 89 are connected to the filing device 76 through the Ethernet, and to the movie recorder 77 through the IEEE1394 bus.

The configuration shown in Fig.14 allows a plurality of medical equipments to share the peripheral equipments 83. Furthermore, because only the endoscope processor 73 has to be turned on for making the endoscopic diagnosis, this configuration contributes to reducing electric power consumption. Note that the peripheral equipment control unit 74 may be a separate body form the endoscope processor 73. It is possible to provide the keyboard 80 with image recording switches, a recording priority selection switch, a display mode switch and a display mode dial, like the embodiment shown in Fig.12, to get the same effects as above.

The connection between the processors and the monitors or the common monitor is not limited to the DVI, but the analog RGB format is usable. Also S-VIDEO format or VIDEO format may also be usable, though the image quality is inferior to the DVI and the analog RGB format.

Although the above embodiments use wired serial buses, like the USB, the Ethernet and the IEEE1394 bus, it is possible to use radio serial buses, such as Bluetooth, IEEE801.11a/b/g.

Thus the present invention is not to be limited to the above-described embodiments, but various modifications will be possible without departing from the scope of claims as appended hereto.

## Claims

1. An ultrasonic endoscope system comprising:
an ultrasonic endoscope (10) having an end portion with an imaging device (12) and an ultrasonic transducer (13), said imaging device (12) capturing an optical image of a certain internal body part to output an image signal, and said ultrasonic transducer (13) emitting an ultrasonic wave toward said internal body part to receive an echo signal from said internal body part;
an endoscope processor (14) for producing an endoscopic image from said image signal;
an ultrasonograph processor (15) for producing an ultrasonographic image from said echo signal; and
a plurality of peripheral equipments (19) that exchange data with said processors;
**characterised in that**
the ultrasonic endoscope system further comprises an observation device (11) comprising said processors (14, 15) and said plurality of peripheral equipments (19), wherein said processors (14, 15) are connected to said peripheral equipments (19) through serial buses having bus arbitration function.

2. An ultrasonic endoscope system as claimed in claim 1, further comprising a peripheral equipment control unit (16) that is connected to said processors and said peripheral equipments, to serve as an intermediary of data between said processors, on one hand, and said peripheral equipments, on the other hand.

3. An ultrasonic endoscope system as claimed in claim 2, wherein said peripheral equipment control unit (16) is connected to said processors and said peripheral equipments through universal serial buses.

4. An ultrasonic endoscope system as claimed in claim 3, wherein said peripheral equipment control unit (16) controls those of said universal serial buses which are connected respectively to said processors, independently from each other.

5. An ultrasonic endoscope system as claimed in claim 4, wherein said peripheral equipment control unit (16) comprises at least two USB controllers (31, 32) and a CPU (30) for controlling operation of said USB controllers, and operates as a host while exchanging data with said peripheral equipments, and as a device while exchanging data with said processors.

6. An ultrasonic endoscope system as claimed in claim 2, wherein said peripheral equipment control unit is incorporated into said endoscope processor.

7. An ultrasonic endoscope system as claimed in claim 1, wherein said peripheral equipments (19) include a movie recorder (26) for recording a selected one of the endoscopic image and the ultrasonographic image as a movie image.

8. An ultrasonic endoscope system as claimed in claim 7, wherein said peripheral equipments (19) include a first operational device (40,41) for selecting one of the endoscopic image and the ultrasonographic image, to be recorded by said movie recorder.

9. An ultrasonic endoscope system as claimed in claim 8, wherein said peripheral equipments include a second operational device (42) for selecting between an endoscope priority mode for giving priority to the movie recording of the endoscopic image and an ultrasonograph priority mode for giving priority to the movie recording of the ultrasonographic image, and wherein said processors control said movie recorder to record the endoscopic image or the ultrasonographic image in accordance with the selection done by said second operational device, prior to the selection done by said first operational device.

10. An ultrasonic endoscope system as claimed in claim 7 , wherein one of said serial buses which connects said processors to said movie recorder is an IEEE1394 bus.

11. An ultrasonic endoscope system as claimed in claim 10, wherein an output plug and an input plug of said IEEE1394 bus are established at a first one of said processors, a second output plug of said IEEE1394 bus is established at a second one of said processors, and a second input plug of said IEEE1394 bus is established at said movie recorder, and wherein a first isochronous channel is established between said first processor and said movie recorder, and a second isochronous channel is established between said first and second processors.

12. An ultrasonic endoscope system as claimed in claim 1, further comprising an image composing device (54) for composing the endoscopic image and the ultrasonographic image to produce a composite image; and
an image display device (51) for displaying a selected one of the endoscopic image, the ultrasonographic image and the composite image.

13. An ultrasonic endoscope system as claimed in claim 12, wherein said peripheral equipments include a third operational device (60,61) for selecting one of the endoscopic image, the ultrasonographic image and the composite image, to be displayed on said image display device.

14. An ultrasonic endoscope system as claimed in claim 12, wherein said image composing device (54) can change the ratio of an endoscopic image display area to an ultrasonographic image display area in the composite image on said image display device (51).

15. An ultrasonic endoscope system as claimed in claim 14, wherein said peripheral equipments include a fourth operational device (61) for causing said image composing device (54) to change the ratio of an endoscopic image display area to an ultrasonographic image display area in the composite image.

16. An ultrasonic endoscope system as claimed in claim 1, wherein at least one of said serial buses is a radio serial bus.

17. An electronic endoscope system comprising :
an electronic endoscope (71) having an end portion with an imaging device (72) for capturing an optical image of a certain internal body part to output an image signal;
an endoscope processor (73) for producing an endoscopic image from said image signal; and
a plurality of peripheral equipments (83) that exchange data with said processors,
wherein said peripheral equipments include at least a second processor (86, 89) that produces from an electric signal an image served for medical diagnosis;
**characterised in that**
said second processor includes an ultrasonograph processor (86) for producing an ultrasonographic image from an echo signal, wherein said endoscope processor is connected to said peripheral equipments, including said ultrasonograph processor (86), through serial buses having bus arbitration function.

18. An electronic endoscope system as claimed in claim 17, further comprising a peripheral equipment control unit (74) connected to said endoscope processor and said peripheral equipments, to serve as an intermediary of data between said endoscope processor and said peripheral equipments (83).

19. An electronic endoscope system as claimed in claim 18, wherein said peripheral equipment control unit (74) is connected to said endoscope processor and said peripheral equipments through universal serial buses.

20. An electronic endoscope system as claimed in claim 18, wherein said peripheral equipment control unit (74) is incorporated into said endoscope processor (73).

21. An electronic endoscope system as claimed in claim 17, wherein said peripheral equipments include a movie recorder (77) for recording the endoscopic image as a movie image.

22. An electronic endoscope system as claimed in claim 21, wherein one of said serial buses which connects said endoscope processor to said movie recorder (77) is an IEEE1394 bus.

23. An electronic endoscope system as claimed in claim 17, wherein said second processor is connected to said movie recorder through a serial bus.

24. An electronic endoscope system as claimed in claim 17, wherein at least one of said serial buses is a radio serial bus.

25. An electronic endoscope system as claimed in claim 17, wherein said second processor includes an optical coherent tomography processor (89) for producing an optical coherent tomographic image.

## Patentansprüche

1. Ultraschallendoskopsystem, umfassend:
ein Ultraschallendoskop (10), das einen Endabschnitt mit einer bildgebenden Einrichtung (12) und einem Ultraschallwandler (13) aufweist, wobei die bildgebende Einrichtung (12) ein optisches Bild eines gewissen inneren Körperteils zum Ausgeben eines Bildsignals aufnimmt und der Ultraschallwandler (13) eine Ultraschallwelle in Richtung des inneren Körperteils emittiert, um von dem inneren Körperteil ein Echosignal zu empfangen;
einen Endoskopprozessor (14) zum Erzeugen eines endoskopischen Bilds aus dem Bildsignal;
einen Echograph-Prozessor (15) zum Erzeugen eines echographischen Bilds aus dem Echosignal; und
eine Mehrzahl von Peripheriegeräten (19), die Daten mit den Prozessoren austauschen,
**dadurch gekennzeichnet, dass** das Ultraschallendoskopsystem außerdem eine Betrachtungseinrichtung (11) aufweist, welche die Prozessoren (14, 15) und die mehreren Peripheriegeräte (19) umfasst, wobei die Prozessoren (14, 15) mit den Peripheriegeräten (19) über serielle Busse mit Busentscheiderfunktion angeschlossen sind.

2. System nach Ansrpuch 1, weiterhin umfassend eine Peripheriegeräte-Steuereinheit (16), die an die Prozessoren und die mehreren Peripheriegeräte angeschlossen ist, um als Datenvermittler zwischen den Prozessoren einerseits und den Peripheriegeräten andererseits zu fungieren.

3. System nach Anspruch 2, bei dem die Peripheriegeräte-Steuereinheit (16) an die Prozessoren und an die Peripheriegeräte über serielle Universalbusse angeschlossen ist.

4. System nach Anspruch 3, bei dem die Peripheriegeräte-Steuereinheit (16) solche von den seriellen Universalbussen, die an die Prozessoren angeschlossen sind, unabhängig voneinander steuert.

5. System nach Anspruch 4, bei dem die Peripheriegeräte-Steuereinheit (16) mindestens zwei USB-Steuerungen (31, 32) und eine CPU (30) zum Steuern des Betriebs der USB-Steuerungen aufweist und als Host arbeitet beim Austausch von Daten mit den Peripheriegeräten, und als Gerät beim Austausch von Daten mit den Prozessoren arbeitet.

6. System nach Anspruch 2, bei dem die Peripheriegeräte-Steuereinheit in den Endoskopprozessor einezogen ist.

7. System nach Anspruch 1, bei dem die Peripheriegeräte (19) einen Filmrekorder (26) zum Aufzeichnen eines ausgewählten Bilds von dem endoskopischen Bild und dem echographischen Bild in Form eines Filmbilds enthält.

8. System nach Anspruch 7, bei dem die Peripheriegeräte (19) eine erste Betätigungseinrichtung (40, 41) zum Auswählen eines von dem endoskopischen Bild und dem echographischen Bild für die Aufzeichnung durch den Filmrekorder enthalten.

9. System nach Anspruch 8, bei dem die Peripheriegeräte eine zweite Bedieneinrichtung (42) zum Auswählen zwischen einem Endoskop-Prioritätsmodus, der der Filmaufzeichnung des endoskopischen Bilds Priorität einräumt, und einem echographischen Prioritätsmodus, der der Filmaufzeichnung des echographischen Bilds Priorität einräumt, enthalten, wobei die Prozessoren den Filmrekorder zum Aufzeichnen des endoskopischen Bilds oder des echographischen Bilds nach Maßgabe der Auswahl seitens der zweiten Bedieneinrichtung steuern, bevor die Auswahl durch die erste Bedieneinrichtung erfolgt.

10. System nach Anspruch 7, bei dem einer der seriellen Busse, welcher die Prozessoren mit dem Filmrekorder verbindet, ein IEEE1394-Bus ist.

11. System nach Anspruch 10, bei dem ein Ausgangsstecker und ein Eingangsstecker des IEEE1394-Busses an einem ersten der Prozessoren eingerichtet sind, und ein zweiter Ausgangsstecker des IEEE1394-Busses an einem zweiten der Prozessoren eingerichtet ist, und ein zweiter Eingangsstecker des IEEE1394-Busses in dem Filmrekorder eingerichtet ist, wobei ein erster isochroner Kanal zwischen dem ersten Prozessor und dem Filmrekorder eingerichtet ist und ein zweiter isochroner Kanal zwischen dem ersten und dem zweiten Prozessor eingerichtet ist.

12. System nach Anspruch 1, weiterhin umfassend eine Bildzusammensetzungseinrichtung (54) zum Zusammensetzen des endoskopischen Bilds und des echographischen Bilds, um ein zusammengesetztes Bild zu erzeugen; und
eine Bildanzeigeeinrichtung (51) zum Anzeigen eines ausgewählten Bilds von dem endoskopischen Bild, dem echographischen Bild und dem zusammengesetzten Bild.

13. System nach Anspruch 12, bei dem die Peripheriegeräte eine dritte Bedieneinrichtung (60, 61) zum Auswählen eines von dem endoskopischen Bild, dem echographischen Bild und dem zusammengesetzten Bild für die Anzeige auf der Bildanzeigeeinrichtung enthalten.

14. System nach Anspruch 12, bei dem die Bildzusammensetzungseinrichtung (54) das Verhältnis der Anzeigefläche für ein endoskopisches Bild zu der Anzeigefläche für ein echographisches Bild innerhalb des zusammengesetzten Bilds auf der Bildanzeigeeinrichtung (51) ändern kann.

15. System nach Anspruch 14, bei dem die Peripheriegeräte eine vierte Bedieneinrichtung (61) enthalten, um die Bildzusammensetzungseinrichtung (54) zu veranlassen, das Verhältnis der Anzeigefläche für ein endoskopisches Bild zu einer Anzeigefläche für ein echographisches bild innerhalb des zusammengesetzten Bilds zu ändern.

16. System nach Anspruch 1, bei dem mindestens einer der seriellen Busse ein serieller Funk-Bus ist.

17. Elektronisches Endoskopsystem, umfassend:
ein elektronisches Endoskop (71), das einen Endabschnitt mit einer bildgebenden Einrichtung (72) zum Aufnehmen eines optischen Bilds eines gewissen inneren Körperteils und zum Ausgeben eines Bildsignals aufweist;
einen Endoskopprozessor (73) zum Erzeugen eines endoskopischen Bilds aus dem Bildsignal; und
eine Mehrzahl von Peripheriegeräten (63), welche Daten mit den Prozessoren austauschen, wobei die Peripheriegeräte mindestens einen zweiten Prozessor (86, 89) enthalten, der aus einem elektrischen Signal ein der medizinischen Diagnose dienendes Bild erzeugt;
**dadurch gekennzeichnet, dass** der Prozessor einen Echographen-Prozessor (86) zum Erzeugen eines echographischen Bilds aus einem Echosignal enthält, wobei der Endoskopprozessor mit den Peripheriegeräten einschließlich des Echographen-Prozessors (86) über serielle Busse mit Busentscheiderfunktion angeschlosen ist.

18. System nach Anspruch 17, weiterhin umfassend eine Peripheriegeräte-Steuereinheit (74), die an den Endoskopprozessor und die Peripheriegeräte angeschlossen ist, um als Datenvermittler zwischen den Endoskopprozessor und den Peripheriegeräten (83) zu fungieren.

19. System nach Anspruch 18, bei dem die Peripheriegeräte-Steuereinheit (74) an dem Endoskopprozessor und die Peripheriegeräte über serielle Universalbusse angeschlossen ist.

20. System nach Anspruch 18, bei dem die Peripheriegeräte-Steuereinheit (74) in den Endoskopprozessor (73) inkorporiert ist.

21. System nach Anspruch 17, bei dem die Peripheriegeräte einen Filmrekorder (77) zum Aufzeichnen des endoskopischen Bilds als beweglichen Film enthalten.

22. System nach Anspruch 21, bei dem einer der seriellen Busse, der den Endoskopprozessor mit dem Filmrekorder (77) verbindet, ein IEEE1394-Bus ist.

23. System nach Anspruch 17, bei dem der zweite Prozessor mit dem Filmrekorder über einen seriellen Bus verbunden ist.

24. System nach Anspruch 17, bei dem mindestens einer der seriellen Busse ein serieller Funkbus ist.

25. System nach Anspruch 17, bei dem der zweite Prozessor einen optischen kohärenten Tomographieprozessor (89) zum Erzeugen eines optischen kohärenten Tomographiebilds enthält.

## Revendications

1. Système d'endoscope à ultrasons comprenant :
un endoscope à ultrasons (10) comportant une partie extrémité pourvue d'un dispositif d'imagerie (12) et d'un transducteur à ultrasons (13), ledit dispositif d'imagerie (12) capturant une image optique d'une certaine partie corporelle interne pour sortir un signal d'image, et ledit transducteur à ultrasons (13) émettant une onde ultrasonore vers ladite partie corporelle interne pour recevoir un signal d'écho provenant de ladite partie corporelle interne ;
un processeur d'endoscope (14) destiné à produire une image endoscopique à partir dudit signal d'image ;
un processeur d'échographe (15) destiné à produire une image échographique à partir du signal d'écho ; et
une pluralité d'équipements périphériques (19) qui échangent des données avec lesdits processeurs ;
**caractérisé en ce que**
le système d'endoscope à ultrasons comprend en outre un dispositif d'observation (11) comprenant lesdits processeurs (14, 15) et ladite pluralité d'équipements périphériques (19), lesdits processeurs (14, 15) étant connectés auxdits équipements périphériques (19) par l'intermédiaire de bus série ayant une fonction d'arbitrage de bus.

2. Système d'endoscope à ultrasons selon la revendication 1, comprenant en outre une unité de commande (16) d'équipement périphérique qui est connectée auxdits processeurs et auxdits équipements périphériques, pour servir d'intermédiaire de données entre lesdits processeurs, d'une part, et lesdits équipements périphériques, d'autre part.

3. Système d'endoscope à ultrasons selon la revendication 2, dans lequel ladite unité de commande (16) d'équipement périphérique est connectée auxdits processeurs et auxdits équipements périphériques par l'intermédiaire de bus série universels.

4. Système d'endoscope à ultrasons selon la revendication 3, dans lequel ladite unité de commande (16) d'équipement périphérique commande lesdits bus série universels qui sont connectés respectivement auxdits processeurs, indépendamment les uns des autres

5. Système d'endoscope à ultrasons selon la revendication 4, dans lequel ladite unité de commande (16) d'équipement périphérique comprend au moins deux dispositifs de commande USB (31, 32) et une unité centrale (30) pour commander le fonctionnement desdits dispositifs de commande USB, et sert d'hôte pendant l'échange de données avec lesdits équipements périphériques, et de dispositif pendant l'échange de données avec lesdits processeurs.

6. Système d'endoscope à ultrasons selon la revendication 2, dans lequel ladite unité de commande d'équipement périphérique est contenue dans ledit processeur d'endoscope.

7. Système d'endoscope à ultrasons selon la revendication 1, dans lequel lesdits équipements périphériques (19) comprennent un enregistreur de film (26) destiné à enregistrer une image sélectionnée parmi l'image endoscopique et l'image échographique en tant qu'image de film.

8. Système d'endoscope à ultrasons selon la revendication 7, dans lequel lesdits équipements périphériques (19) comprennent un premier dispositif fonctionnel (40, 41) pour sélectionner l'image endoscopique ou l'image échographique, à enregistrer par ledit enregistreur de film.

9. Système d'endoscope à ultrasons selon la revendication 8, dans lequel lesdits équipements périphériques comprennent un deuxième dispositif fonctionnel (42) destiné à effectuer une sélection entre un mode priorité à l'endoscope pour donner la priorité à l'enregistrement de film de l'image endoscopique et un mode priorité à l'échographe pour donner la priorité à l'enregistrement de film de l'image échographique, et dans lequel lesdits processeurs commandent ledit enregistreur de film afin d'enregistrer l'image endoscopique ou l'image échographique conformément à la sélection effectuée par ledit deuxième dispositif fonctionnel, avant la sélection effectuée par ledit premier dispositif fonctionnel.

10. Système d'endoscope à ultrasons selon la revendication 7, dans lequel l'un desdits bus série qui connecte lesdits processeurs audit enregistreur de film est un bus IEEE1394.

11. Système d'endoscope à ultrasons selon la revendication 10, dans lequel une fiche de sortie et une fiche d'entrée dudit bus IEEE1394 sont établies au niveau d'un premier desdits processeurs, une seconde fiche de sortie dudit bus IEEE1394 est établie au niveau d'un second desdits processeurs, et une seconde fiche d'entrée dudit bus IEEE1394 est établie au niveau dudit enregistreur de film, et dans lequel un premier canal isochrone est établi entre ledit premier processeur et ledit enregistreur de film, et un second canal isochrone est établi entre lesdits premier et second processeurs.

12. Système d'endoscope à ultrasons selon la revendication 1, comprenant en outre un dispositif de composition d'image (54) destiné à composer l'image endoscopique et l'image échographique afin de produire une image composite ; et
un dispositif d'affichage d'image (51) destiné à afficher une image sélectionnée parmi l'image endoscopique, l'image échographique et l'image composite.

13. Système d'endoscope à ultrasons selon la revendication 12, dans lequel lesdits équipements périphériques comprennent un troisième dispositif fonctionnel (60, 61) destiné à sélectionner une image parmi l'image endoscopique, l'image échographique et l'image composite, à afficher sur ledit dispositif d'affichage d'image.

14. Système d'endoscope à ultrasons selon la revendication 12, dans lequel ledit dispositif de composition d'image (54) peut modifier le rapport zone d'affichage d'image endoscopique sur zone d'affichage d'image échographique dans l'image composite sur ledit dispositif d'affichage d'image (51).

15. Système d'endoscope à ultrasons selon la revendication 14, dans lequel lesdits équipements périphériques comprennent un quatrième dispositif fonctionnel (61) destiné à amener ledit dispositif de composition d'image (54) à modifier le rapport zone d'affichage d'image endoscopique sur zone d'affichage d'image échographique dans l'image composite.

16. Système d'endoscope à ultrasons selon la revendication 1, dans lequel au moins l'un desdits bus série est un bus série radio.

17. Système d'endoscope électronique comprenant :
un endoscope électronique (71) comportant une partie extrémité pourvue d'un dispositif d'imagerie (72) destiné à capturer une image optique d'une certaine partie corporelle interne pour sortir un signal d'image ;
un processeur d'endoscope (73) destiné à produire une image endoscopique à partir dudit signal d'image ; et
une pluralité d'équipements périphériques (83) qui échangent des données avec lesdits processeurs ;
dans lequel lesdits équipements périphériques comprennent au moins un second processeur (86, 89) qui produit, à partir d'un signal électrique, une image utilisée pour un diagnostic médical ;
**caractérisé en ce que**
ledit second processeur comprend un processeur d'échographe (86) destiné à produire une image échographique à partir d'un signal d'écho,
dans lequel ledit processeur d'endoscope est connecté auxdits équipements périphériques, comprenant ledit processeur d'échographe (86), par l'intermédiaire de bus série ayant une fonction d'arbitrage de bus.

18. Système d'endoscope électronique selon la revendication 17, comprenant en outre une unité de commande (74) d'équipement périphérique connectée audit processeur d'endoscope et auxdits équipements périphériques, pour servir d'intermédiaire de données entre ledit processeur d'endoscope et lesdits équipements périphériques (83).

19. Système d'endoscope électronique selon la revendication 18, dans lequel ladite unité de commande (74) d'équipement périphérique est connectée audit processeur d'endoscope et auxdits équipements périphériques par l'intermédiaire de bus série universels.

20. Système d'endoscope électronique selon la revendication 18, dans lequel ladite unité de commande (74) d'équipement périphérique est contenue dans ledit processeur d'endoscope (73).

21. Système d'endoscope électronique selon la revendication 17, dans lequel lesdits équipements périphériques comprennent un enregistreur de film (77) destiné à enregistrer l'image endoscopique en tant qu'image de film.

22. Système d'endoscope électronique selon la revendication 21, dans lequel l'un desdits bus série qui connecte ledit processeur d'endoscope audit enregistreur de film est un bus IEEE1394.

23. Système d'endoscope électronique selon la revendication 17, dans lequel ledit second processeur est connecté audit enregistreur de film par l'intermédiaire d'un bus série.

24. Système d'endoscope électronique selon la revendication 17, dans lequel au moins l'un desdits bus série est un bus série radio.

25. Système d'endoscope électronique selon la revendication 17, dans lequel ledit second processeur comprend un processeur de tomographie cohérent optique (89) destiné à produire une image tomographique cohérente optique.
